# EUROPEAN PATENT APPLICATION

(11) **EP 4 744 576 A1**
(43) Date of publication of application: **20.05.2026**
(21) Application number: 23948394.4
(22) Date of filing: 07.08.2023
(51) Int. Cl.: A61B 1/12

(54) **ENDOSCOPE REPROCESSOR, METHOD FOR OPERATING ENDOSCOPE REPROCESSOR, AND PROGRAM FOR ENDOSCOPE REPROCESSOR**

(71) Applicant: Olympus Medical Systems Corp., Tokyo 192-8507 (JP)
(72) Inventor: MOCHIZUKI Tsuyoshi, Hachioji-shi, Tokyo 192-8507 (JP); SEKINO Kohei, Hachioji-shi, Tokyo 192-8507 (JP)
(74) Representative: Seemann & Partner Patentanwälte mbB
(86) International application number: PCT/JP2023/028682
(87) International publication number: WO 2025/032674

(57) **Abstract**

An endoscope reprocessor includes a processing tank in which an endoscope is to be placed, a bottle in which a liquid is to be contained, a conduit that supplies the liquid in the bottle into the processing tank, a pump disposed in the conduit, a flowmeter that measures a flow rate of the liquid, and a controller that detects a shortage of the liquid in the bottle based on a decrease in the flow rate measured by the flowmeter.

## Description

### Technical Field

The present disclosure relates to an endoscope reprocessor, a method of operating the endoscope reprocessor, and a program for the endoscope reprocessor.

### Background Art

For endoscopes used in the medical field, reprocessing processing, such as cleaning and disinfection, is essential for reuse after an insertion portion is inserted into a body for intra-body observation and treatment is performed with a treatment instrument. Endoscope reprocessors are used to perform the reprocessing processing safely, reliably, and automatically.

Japanese Patent Application Laid-Open Publication No. 2017-23406 discloses an endoscope reprocessor in which, when it is detected that a first bottle containing a medicinal solution to be supplied into a processing tank is empty, the medicinal solution is supplied from a second bottle.

A flowmeter for measuring a supply amount of the medicinal solution can be used to detect that the bottle is empty when the flow rate of the medicinal solution is less than or equal to a predetermined flow rate (threshold value).

However, the change in viscosity with respect to temperature varies depending on the type of the medicinal solution. For this reason, as mentioned below, using a flowmeter whose flow rate measurements are affected by viscosity may result in a delay in detecting that the bottle is empty. The delay in detecting that the bottle is empty causes air to be mixed into the cleaning solution, thus making it impossible to supply a predetermined amount of medicinal solution to the processing tank.

If an energizing section dedicated for detection is disposed instead of a flowmeter in order to detect that the bottle is empty, the apparatus becomes complicated and more expensive.

### Citation List

### Patent Literature

Patent Literature 1: Japanese Patent Application Laid-Open Publication No. 2017-23406

### Summary of the Invention

### Problem to be Solved by the Invention

An objective of the present embodiment is to provide an endoscope reprocessor of simple configuration capable of supplying a predetermined amount of liquid into a processing tank, a method of operating the endoscope reprocessor of simple configuration capable of supplying the predetermined amount of liquid into the processing tank, and a program for the endoscope reprocessor of simple configuration capable of supplying the predetermined amount of liquid into the processing tank.

### Means for Solving the Problem

An endoscope reprocessor according to an embodiment of the present disclosure includes a processing tank in which an endoscope is to be placed, a bottle in which a liquid is to be contained, a conduit that supplies the liquid in the bottle into the processing tank, a pump disposed in the conduit, a flowmeter that measures a flow rate of the liquid, and a controller that detects a shortage of the liquid in the bottle based on a decrease in the flow rate measured by the flowmeter.

A method of operating an endoscope reprocessor according to an embodiment of the present disclosure includes detecting a shortage of a liquid in a bottle based on a decrease in a flow rate at which the liquid is supplied into a processing tank by a pump, the liquid being in the bottle in which the liquid is to be contained, the processing tank being for placement of an endoscope.

A program for an endoscope reprocessor according to an embodiment of the present disclosure causes a computer to detect a shortage of a liquid in a bottle based on a decrease in a flow rate at which the liquid is supplied into a processing tank by a pump, the liquid being in the bottle in which the liquid is to be contained, the processing tank being for placement of an endoscope.

### Effects of the Invention

An embodiment of the present disclosure can provide an endoscope reprocessor of simple configuration capable of supplying a predetermined amount of liquid into a processing tank, a method of operating the endoscope reprocessor of simple configuration capable of supplying the predetermined amount of liquid into the processing tank, and a program for the endoscope reprocessor of simple configuration capable of supplying the predetermined amount of liquid into the processing tank.

### Brief Description of the Drawings

Fig. 1 is a perspective view of an endoscope reprocessor of an embodiment.
Fig. 2 is a configuration diagram of a liquid supply unit of the endoscope reprocessor of the embodiment.
Fig. 3 is a flowchart of a method of operating the endoscope reprocessor of the embodiment.
Fig. 4 is a configuration diagram of a liquid supply unit showing a state of feeding a liquid in a state where a bottle of the endoscope reprocessor of the embodiment is empty.
Fig. 5 is a view showing a change in the number of rotations of a flowmeter while a cleaning solution 1 is being supplied.
Fig. 6 is a view showing a change in the number of rotations of the flowmeter while a cleaning solution 2 is being supplied.

### Modes for Carrying Out the Invention

### <First Embodiment>

An endoscope reprocessor 1 of the present embodiment will be described using the drawings. In the following, the endoscope reprocessor 1 will be referred to as a reprocessor 1.

### <Overall Configuration of Reprocessor>

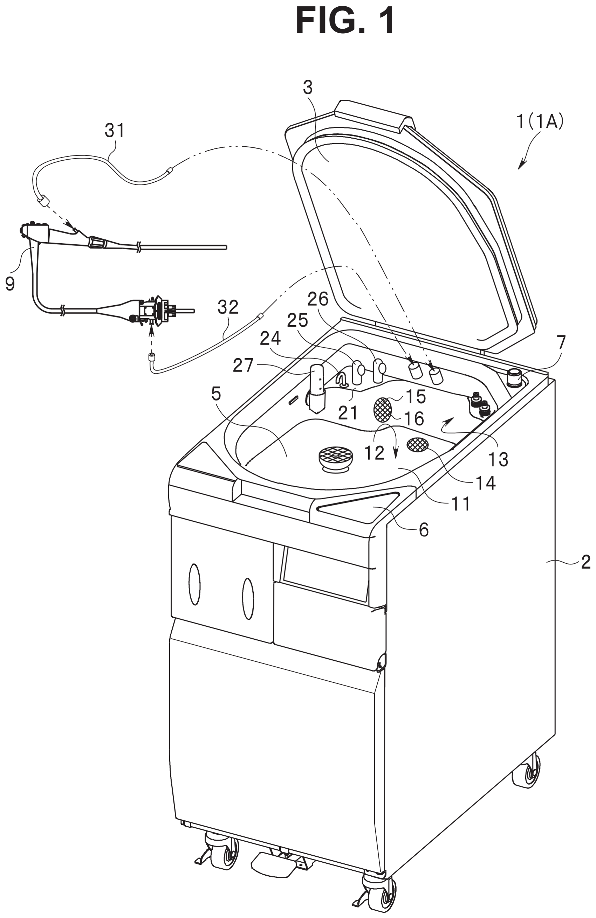

As shown in Fig. 1, the reprocessor 1 includes a main body 2 and a top cover 3 which is openable and closable. Fig. 1 shows a state where the top cover 3 of the reprocessor 1 is open.

The reprocessor 1 is an apparatus for performing reprocessing processing (restoration processing) on an endoscope 9 or an endoscope accessory. The reprocessing processing may be rinsing with water, cleaning to remove contaminants such as organic matter, disinfection to inactivate predetermined microorganisms, sterilization to eliminate or kill all microorganisms, or any combination of these.

The main body 2 includes, at the top thereof, a processing tank 5 for performing processing such as cleaning and disinfection of the endoscope 9, an operation panel 6, and a water-supply-hose connection port 7.

The processing tank 5 stores a liquid such as cleaning solution, water, alcohol, disinfectant solution, or sterilization solution. The processing tank 5 includes an endoscope placement section 11 and a terrace 21.

The endoscope placement section 11 includes a bottom surface 12 and a side surface 13, is capable of placing the endoscope 9, and stores the liquid. A discharge port 14 for discharging the stored liquid is provided on the bottom surface 12 of the endoscope placement section 11. A circulation port 16 having a mesh filter 15 is provided on the side surface 13 of the endoscope placement section 11. The circulation port 16 is in communication with a circulation pump. The circulation port 16 may be provided on the bottom surface 12.

The terrace 21 is provided at a position adjacent to the endoscope placement section 11 and higher than the endoscope placement section 11. The terrace 21 includes a water supply port 22, a gas feeding port 23, a cleaning solution nozzle 24, a disinfectant solution nozzle 25, a water supply nozzle 26, and a water level sensor 27.

The water supply port 22 is a port to which a first tube 31 is to be connected. The gas feeding port 23 is a port to which a second tube 32 is to be connected. The number of ports that the reprocessor 1 has is not limited to 2.

The cleaning solution nozzle 24 supplies a cleaning solution L into the processing tank 5 by a cleaning-solution supply unit 10 mentioned below. The disinfectant solution nozzle 25 supplies a disinfectant solution into the processing tank 5. The water supply nozzle 26 supplies water taken in from the water-supply-hose connection port 7 into the processing tank 5, and also supplies, into the processing tank 5 again, the liquid in the processing tank 5 taken in from the circulation port 16 having the mesh filter 15, thereby circulating the liquid. The mesh filter 15 filters contaminants P from the liquid. The water level sensor 27 detects a water level of the liquid stored in the processing tank 5.

The operation panel 6 is placed at an upper front section of the main body 2. The operation panel 6 includes various operation buttons, not shown. A user uses the operation panel 6 to give various instructions to the reprocessor 1.

The water-supply-hose connection port 7 is provided at an upper rear section of the main body 2. A water supply hose connected to a water faucet, not shown, is connected to the water-supply-hose connection port 7, and water is supplied into the reprocessor 1 via the water supply nozzle 26.

The top cover 3 is provided openably and closably on the top of the main body 2. In the reprocessor 1, by bringing the top cover 3 into an opened state, the endoscope 9 can be placed on the endoscope placement section 11, and connection between the endoscope 9 and the reprocessor 1 can be established by means of the first tube 31 and the second tube 32. The reprocessor 1 is in a state where reprocessing processing is possible by bringing the top cover 3 into a closed state after the endoscope 9 is set.

### <Cleaning-Solution Supply Unit>

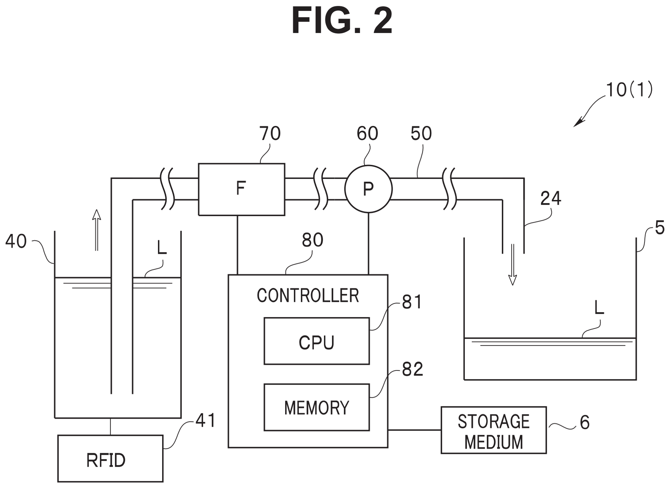

As shown in Fig. 2, the cleaning-solution supply unit 10 includes a bottle 40, a conduit 50, a pump 60, a flowmeter 70, and a controller (processor) 80.

The bottle 40 can have any shape that can contain the cleaning solution L, such as, for example, a cylindrical shape or a square tubular shape. The cleaning solution L may be a concentrated solution that is diluted with water for use in the processing tank. Once the filled cleaning solution L has been emptied through use, the bottle 40 is removed from the endoscope reprocessor 1 and replaced with a new bottle 40 filled with the cleaning solution L. The conduit 50 configures a pathway to supply the cleaning solution L in the bottle 40 into the processing tank.

The pump 60 disposed in the conduit 50 suctions the cleaning solution L in the bottle 40 and supplies the solution into the processing tank 5. The pump 60 is a tube pump, for example. The tube pump is a metering pump and has a small flow rate change due to pulsation, and is therefore particularly suitable for the reprocessor 1 of the present embodiment.

The flowmeter 70 measures the flow rate of the cleaning solution L through the conduit 50 at a predetermined time interval ΔT (e.g., 1 second). The flowmeter 70 may preferably be of a rotary type in view of compactness and low cost. The flowmeter 70 is a turbine-type flowmeter, for example.

The controller 80 includes a CPU 81, which is a central processing unit of a computer, and a memory 82, which includes a ROM, a RAM, and the like. As mentioned below, the controller 80 detects a shortage of the cleaning solution L in the bottle 40 based on a decrease in the flow rate measured by the flowmeter 70.

Functions of the controller 80 are realized by the CPU81 reading out and executing a program or the like from the memory 82. The program stored in the memory 82 for causing the computer to execute the liquid supply processing is stored on a non-transitory computer-readable storage medium 8, and may be transferred to the memory 82.

The controller 80 that controls the cleaning-solution supply unit 10 may not be dedicated to the cleaning-solution supply unit 10, but may be part of a controller that controls the entire reprocessor 1.

### <Method of Operating Reprocessor>

An example of the method of operating the reprocessor will be briefly described below.

### <Step S10> Endoscope Placement Step

The user opens the top cover 3 of the reprocessor 1 and sets the endoscope 9. After connection is established between the reprocessor 1 and internal conduits of the endoscope 9 using the first tube 31 and the second tube 32, the user places the endoscope 9 on the endoscope placement section 11 and brings the top cover 3 into a closed state.

When the user gives an instruction from the operation panel 6 to start a predetermined processing such as cleaning and disinfection, the CPU81 reads a predetermined program from the memory 82 and starts processing of the program.

### <Step S11> Water Supply Step

Based on a control signal from the CPU81, water is supplied from the water supply nozzle 26 into the processing tank 5. When the water level in the processing tank 5 detected by the water level sensor 27 reaches a predetermined value, the water supply automatically stops.

### <Step S12> Ultrasonic Cleaning Step

When a transducer, not shown, disposed on a lower surface of the processing tank 5 is activated, ultrasonic waves are applied to the water stored in the processing tank 5. Ultrasonic cleaning cleans the contaminants on an outer surface of the endoscope 9.

### <Step S13> Conduit Cleaning Step

The controller 80 supplies water into the internal conduit of the endoscope 9 via the first tube 31 and the second tube 32. Contaminants P in the conduits are discharged from an opening at a distal end of the endoscope 9 along with the water. The water stored in the processing tank 5 is discharged via the circulation port 16 and supplied into the processing tank 5 again.

### <Step S14> Fluid Cleaning Step

The cleaning solution L in the bottle 40 is poured into the processing tank 5, in which water is stored, from the cleaning solution nozzle 24 by the cleaning-solution supply unit 10. A method of operating the cleaning-solution supply unit 10 will be mentioned in detail below.

The cleaning solution L, diluted by the water stored in the processing tank 5, is discharged via the circulation port 16 and supplied into the processing tank 5 again.

### <Step S15> Disinfection Step

After the diluted cleaning solution L is discharged from the processing tank 5, the disinfectant solution connected to a disinfectant solution tank is poured into the processing tank 5 from the disinfectant solution nozzle 25. The disinfectant solution is sucked in from the circulation port 16 and supplied into the processing tank 5 again.

### <Step S 16> Drying Step

After the disinfectant solution is discharged from the processing tank 5, air is fed into a conduit 90 and air-drying processing is performed to remove moisture in the conduit. A drying liquid such as alcohol, not shown, may be fed into the conduit 90.

This completes the reprocessing processing of the endoscope 9 placed in the processing tank 5.

It is noted that the reprocessing processing is not limited to the example mentioned above. For example, a rinsing step using water and the drying step may also be performed between each step, and the conduit cleaning step may be omitted.

### <Method of Operating Cleaning-Solution Supply Unit>

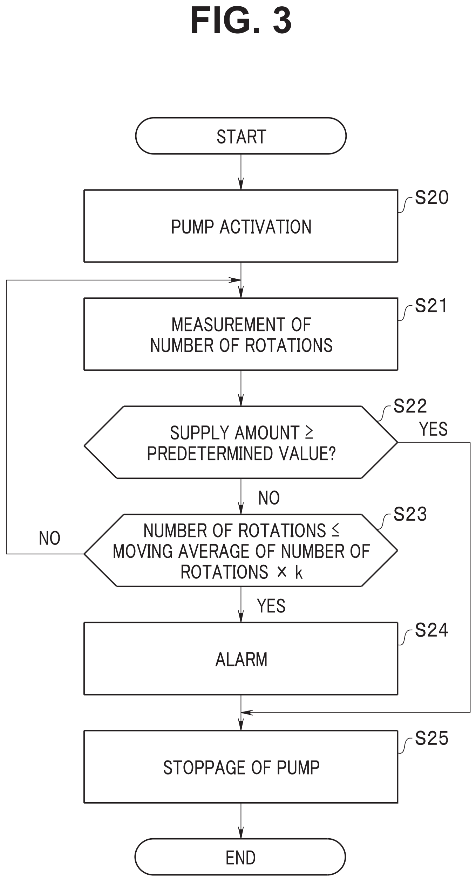

The method of operating the cleaning-solution supply unit will be described along a flowchart in Fig. 3. It is noted that, in the following, the flow rate will be expressed using the number of rotations of the turbine-type flowmeter 70.

### <Step S20> Pump Activation

Upon receiving a control signal from the controller 80, the pump 60 starts feeding the cleaning solution L from the bottle 40 to the processing tank 5. It is noted that the pump 60, which is a tube pump, for example, is a metering pump with a constant number of rotations (flow rate).

### <Step S21> Measurement of Number of Rotations

The flowmeter 70 measures the number of rotations of the cleaning solution L flowing through the conduit 50 at every predetermined time interval ΔT. The interval ΔT is based on the specifications of the flowmeter 70, or the like. The upper limit of the interval ΔT may preferably be 2 seconds or less, and may especially preferably be 1 second or less. If the interval ΔT is less than or equal to the upper limit mentioned above, a desired amount of the cleaning solution L can be supplied accurately. The lower limit of the interval ΔT is not particularly limited, but is 0.1 second, for example.

### <Step S22> Supply Amount ≥ Predetermined Value?

Upon starting feeding the liquid, the controller 80 starts measuring the time, and when a predetermined time elapses, stops the pump 60 to end supplying the cleaning solution L. That is, the processing of supplying the cleaning solution L ends when the amount of the cleaning solution L supplied into the processing tank 5 reaches a predetermined value. The predetermined liquid feeding amount is set as an amount of the cleaning solution L required for one reprocessing processing.

It is noted that the controller 80 may store, in, e.g., the memory 82, a summed-up amount SM (the number of rotations * time), which is obtained by summing up the number of rotations detected by the flowmeter 70. When the summed-up amount SM reaches a predetermined value, the controller 80 controls the cleaning-solution supply unit 10 to end the processing of supplying the cleaning solution L.

### <Step S23> Number of Rotations ≤ Moving Average of Number of Rotations

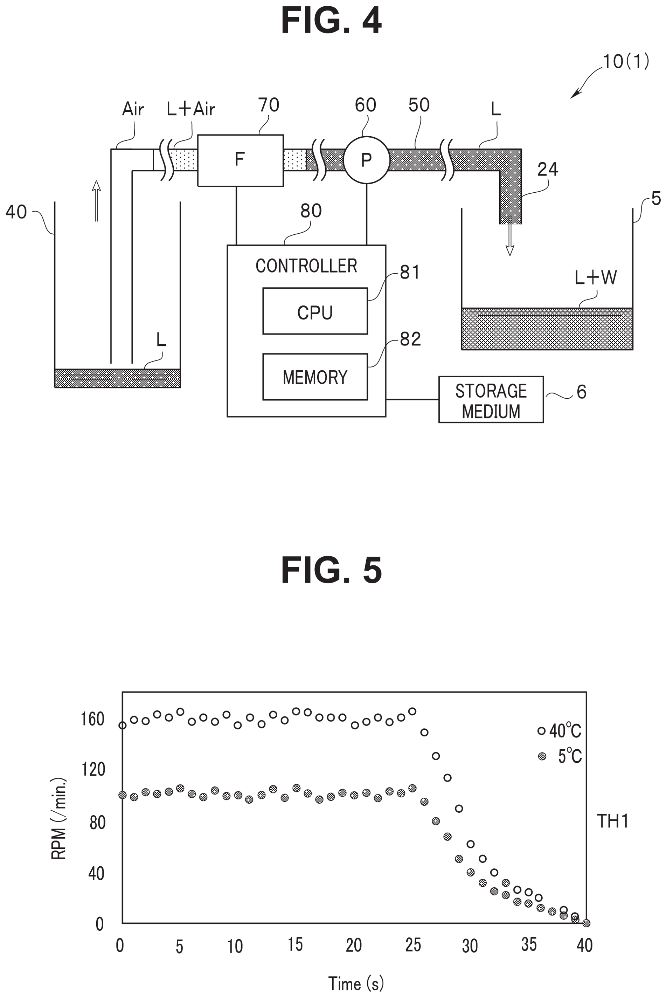

As shown in Fig. 4, when a shortage of the cleaning solution L in the bottle 40 occurs, air is mixed into the cleaning solution L drawn into the conduit 50 (L + Air). An area of the conduit 50 near the cleaning solution nozzle 24 is filled with the cleaning solution L, but only air exists in an area of the conduit 50 near the bottle 40. The flowmeter 70 of the embodiment is a turbine-type flowmeter for liquids, and when gas is mixed into the measured liquid, the number of rotations decreases.

Fig. 5 shows a change in the number of rotations of the flowmeter 70 in a case where a shortage of the cleaning solution L in the bottle 40 occurs at 25 seconds and where the cleaning solution L in the bottle 40 is empty at 40 seconds. When the cleaning solution L in the bottle 40 is brought into a shortage state, air is mixed into the cleaning solution L, and the number of rotations of the flowmeter 70 decreases.

With the cleaning solution L1 shown in Fig. 5, by setting in advance a threshold value TH1, which is a predetermined number of rotations, the controller 80 can detect in a short time that there is a shortage of the cleaning solution L in the bottle 40 with reference to the threshold value TH1. For example, if the threshold value TH1 is set at 80 rpm, the controller 80 can detect a shortage of the cleaning solution L at 27 seconds in the case where the liquid temperature is 5 °C. The controller 80 can also detect a shortage of the cleaning solution L at 30 seconds also in the case where the liquid temperature is 40 °C.

That is, even when the temperature of the cleaning solution L1 is increased from 5 °C to 40 °C, the number of rotations detected by the flowmeter 70 at a standard liquid feeding amount only increases by about 1.6 times, from 100 rpm to 160 rpm. This is because the change in viscosity of the cleaning solution L1 with respect to temperature is substantially the same as the change in viscosity of water with respect to temperature (-0.5 mPa·s/°C). For this reason, with the cleaning solution L1, even if the temperature changes, the controller 80 can detect a shortage of the cleaning solution L within 5 seconds after the flow rate begins to decrease.

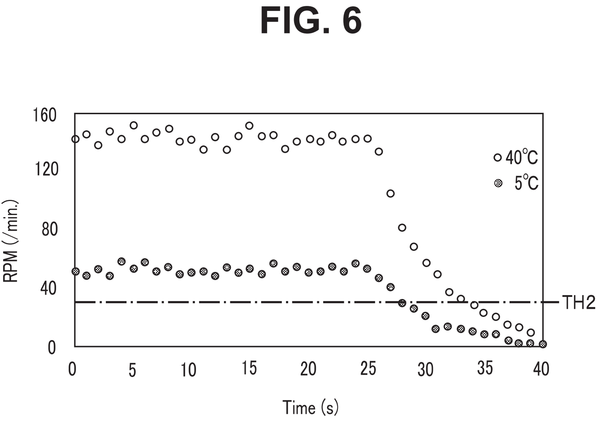

However, with a cleaning solution L2 shown in Fig. 6, when the temperature is increased from 5 °C to 40 °C, the number of rotations detected by the flowmeter at the standard liquid feeding amount increases about three times from 50 rpm to 150 rpm. This is because the change in viscosity of the cleaning solution L2 with respect to temperature is four times greater (-2 mPa·s/°C) than the change in viscosity of water with respect to temperature.

For this reason, if the threshold value is set at 80 rpm for the cleaning solution L2, as in the same manner as for the cleaning solution L1, the controller 80 cannot detect that there is a shortage of the cleaning solution L at 5 °C.

For example, if a threshold value TH2 is set at 30 rpm corresponding to a liquid temperature of 5 °C, then at 40 °C, the time from when the flow rate begins to decrease until the controller 80 detects a shortage of the cleaning solution L ends up being 8 seconds. For the cleaning solution L2, whether the temperature is 5 °C or 40 °C, there is no threshold value at which the controller 80 can detect a shortage of the cleaning solution L within 5 seconds.

The number of rotations detected by the flowmeter 70 varies not only due to temperature, but also due to individual differences (manufacturing errors, deterioration) of the pump 60 or the flowmeter 70, so the conversion coefficient, used when the number of rotations is converted to a flow rate, may change.

That is, in a detection method using the number of rotations as a threshold value, depending on the type of the cleaning solution L, it may take time for the controller 80 to detect a shortage of the cleaning solution L, and the cleaning solution L mixed with air may be fed.

In contrast, in the reprocessor 1, the controller 80 detects a shortage of the cleaning solution L based on a moving average value (moving average of the number of rotations or moving average flow rate) of the most recent N measurements of the number of rotations measured by the flowmeter 70, where N is an integer greater than or equal to 2. The number of times N can be set as desired, but from the standpoint of detection accuracy, may preferably be greater than or equal to 3 and less than or equal to 6, for example.

When the number of rotations is less than a threshold value obtained by multiplying the moving average of the number of rotations by a predetermined coefficient k, the controller 80 detects that there is a shortage of the cleaning solution L. The predetermined coefficient k can be set as desired, but may preferably be less than 0.9 and greater than 0.7. If the coefficient k is less than the aforementioned range, the controller 80 is less likely to make a false detection, and if the coefficient k is greater than the aforementioned range, the controller 80 is not delayed in detecting a shortage of the cleaning solution L.

Hereinafter, a case (number of times N = 3, coefficient k = 0.8) will be described as an example.

In the cleaning solution L1 in Fig. 5, the moving average of the number of rotations at 5 °C and 25 seconds is 102 rpm. The moving average of the number of rotations at 40 °C and 25 seconds is 160 rpm. It takes 27 seconds for the number of rotations at 5 °C to reach (102 * k) rpm, i.e., 82 rpm. It takes 27 seconds for the number of rotations at 40 °C to reach 120 rpm.

In the cleaning solution L2 in Fig. 6, the moving average of the number of rotations at 5 °C and 25 seconds is 52 rpm. The moving average of the number of rotations at 40 °C and 25 seconds is 141 pm. It takes 27 seconds for the number of rotations at 5 °C to reach (52 * k) rpm, i.e., 42 rpm. It also takes 27 seconds for the number of rotations at 40 °C to reach 113 rpm.

According to the controller 80 of the reprocessor 1, a shortage of the cleaning solution L can be detected within 5 seconds after the flow rate begins to decrease, even in the case of a cleaning solution whose viscosity changes significantly with respect to temperature, exceeding twice that of water.

It is noted that, in the reprocessor 1 in which cleaning solutions with different characteristics (model numbers) are used depending on the type or the like of the endoscope to be cleaned, the optimal coefficient k for each cleaning solution may be stored in advance by the controller 80 together with the model number, may be inputted by the user using the operation panel 6, or may be selected from among a plurality of stored model numbers or a plurality of coefficients k.

The bottle 40 may include a non-volatile memory, such as an RFID tag 41, and the controller 80 may obtain the model number or the coefficient k of the cleaning solution from the RFID tag 41.

The coefficient k is set so that a shortage of the cleaning solution L can be detected within 5 seconds when the temperature of the cleaning solution L is in a range from 5 °C to 40 °C.

It is noted that, if no shortage of the cleaning solution L is detected (S23: NO), processings from S21 are repeated.

### <Steps S24 and S25> Alarm and Stoppage of Pump

When detecting a shortage of the cleaning solution L (S22: YES), the controller 80 generates an alarm signal, and stops the pump 60 to stop supplying the cleaning solution L.

The alarm signal is communicated to the user, for example, by displaying an image or text on the operation panel 6, or by generating a buzzer sound. The user replaces the bottle 40, removes the cleaning solution L mixed with air in the conduit 50, and then instructs the reprocessor 1 to resume the processing. It is noted that, in a reprocessor including a plurality of bottles 40, the bottle 40 that supplies the cleaning solution may be automatically altered.

As described above, the method of operating the endoscope reprocessor detects a shortage of the liquid in the bottle in which the liquid is contained, based on a decrease in the flow rate of the liquid supplied by the pump into the processing tank in which the endoscope is placed.

A program for the method of operating the endoscope reprocessor operates a computer to detect a shortage of the liquid in the bottle in which the liquid is contained, based on a decrease in the flow rate of the liquid supplied by the pump into the processing tank in which the endoscope is placed.

### <Modification Example>

An endoscope reprocessor 1A of the modification example is similar to and provides the same effect as the endoscope reprocessor 1 of the embodiment. For this reason, description of the same configuration as that of the endoscope reprocessor 1 of the embodiment will be omitted below.

The controller 80 of the endoscope reprocessor 1A of the modification example may detect a shortage of the cleaning solution L when the number of rotations (flow rate) decreases K consecutive times, where K is a natural number greater than or equal to 2. The number of times K can be set as desired, but from the standpoint of detection accuracy, may preferably be greater than or equal to 3 and less than or equal to 6, for example.

It goes without saying that, even if the same configuration as the cleaning-solution supply unit 10 is used in a liquid supply unit of the endoscope reprocessor, such as a disinfectant-solution supply unit or an alcohol supply unit, the same effect as the cleaning-solution supply unit 10 is provided.

In order to detect mixture of air into the liquid as quickly as possible, it may be preferable that, in the conduit 50, the flowmeter 70 be placed at a position closer to the bottle 40 than the pump 60, as shown in Fig. 2.

The present disclosure is not limited to the embodiments mentioned above, and various alterations, modifications, etc. are possible within the scope not changing the gist of the present disclosure.

### List of Reference Signs

- 1, 1A: endoscope reprocessor
- 2: main body
- 3: top cover
- 5: processing tank
- 6: operation panel
- 8: storage medium
- 9: endoscope
- 10: cleaning-solution supply unit
- 40: bottle
- 41: RFID tag
- 50: conduit
- 60: pump
- 80: controller
- 80: flowmeter
- 81: CPU
- 82: memory
- 90: conduit

## Claims

1. An endoscope reprocessor comprising:
a processing tank in which an endoscope is to be placed;
a bottle in which a liquid is to be contained;
a conduit that supplies the liquid in the bottle into the processing tank;
a pump disposed in the conduit;
a flowmeter that measures a flow rate of the liquid; and
a controller that detects a shortage of the liquid in the bottle based on a decrease in the flow rate measured by the flowmeter.

2. The endoscope reprocessor according to claim 1, wherein the controller calculates a moving average value of the flow rate over N times, where N is an integer greater than or equal to 2, and detects the shortage of the liquid based on the moving average value of the flow rate.

3. The endoscope reprocessor according to claim 2, wherein the controller detects the shortage of the liquid when the flow rate is less than a threshold value obtained by multiplying the moving average value by a predetermined coefficient.

4. The endoscope reprocessor according to claim 3, wherein the controller stores the predetermined coefficient corresponding to each type of the liquid.

5. The endoscope reprocessor according to claim 3, wherein
the bottle includes a memory storing the predetermined coefficient corresponding to each type of the liquid contained in the bottle, and
the controller obtains the predetermined coefficients from the memory.

6. The endoscope reprocessor according to claim 1, wherein the controller detects the shortage of the liquid when the flow rate decreases K consecutive times, where K is a natural number greater than or equal to 2.

7. The endoscope reprocessor according to claim 1, wherein the controller stops the pump and generates an alarm signal when the controller detects the shortage of the liquid.

8. The endoscope reprocessor according to claim 1, wherein
the pump is a tube pump, and
the flowmeter is a turbine-type flowmeter.

9. The endoscope reprocessor according to claim 1, wherein, in the conduit, the flowmeter is disposed at a position closer to the bottle than the pump.

10. The endoscope reprocessor according to claim 1, wherein a change in viscosity of the liquid with respect to temperature exceeds twice the change in viscosity of water with respect to temperature.

11. The endoscope reprocessor according to claim 10, wherein the liquid is a cleaning solution.

12. The endoscope reprocessor according to claim 11, wherein the shortage of the cleaning solution can be detected within 5 seconds when a temperature of the cleaning solution is in a range from 5 °C to 40 °C.

13. A method of operating an endoscope reprocessor, the method comprising:
detecting a shortage of a liquid in a bottle based on a decrease in a flow rate at which the liquid is supplied into a processing tank by a pump, the liquid being in the bottle in which the liquid is to be contained, the processing tank being for placement of an endoscope.

14. The method of operating the endoscope reprocessor according to claim 13, the method further comprising:
calculating a moving average value of the flow rate over N times, where N is an integer greater than or equal to 2, and detects the shortage of the liquid based on the moving average value of the flow rate.

15. The method of operating the endoscope reprocessor according to claim 14, the method further comprising:
detecting the shortage of the liquid when the flow rate is less than a threshold value obtained by multiplying the moving average value by a predetermined coefficient.

16. The method of operating the endoscope reprocessor according to claim 14, the method further comprising:
detecting the shortage of the liquid when the flow rate decreases K consecutive times, where K is a natural number greater than or equal to 2.

17. A program for an endoscope reprocessor, the program causing a computer to detect a shortage of a liquid in a bottle based on a decrease in a flow rate at which the liquid is supplied into a processing tank by a pump, the liquid being in the bottle in which the liquid is to be contained, the processing tank being for placement of an endoscope.

18. The program for the endoscope reprocessor according to claim 17, wherein the program calculates a moving average value of the flow rate over N times, where N is an integer greater than or equal to 2, and detects the shortage of the liquid based on the moving average value of the flow rate.

19. The program for the endoscope reprocessor according to claim 18, wherein the program detects the shortage of the liquid when the flow rate is less than a threshold value obtained by multiplying the moving average value by a predetermined coefficient.

20. The program for the endoscope reprocessor according to claim 17, wherein the program detects the shortage of the liquid when the flow rate decreases K consecutive times, where K is a natural number greater than or equal to 2.
